Europäisches Patentamt

(19) **European Patent Office**

Office européen des brevets

(11) Publication number: **0 224 183**
**B1**

(12) **EUROPEAN PATENT SPECIFICATION**

(45) Date of publication of the patent specification:
28.03.90

(51) Int. Cl.⁴: **C10L 1/32**
// C12P19/00

(21) Application number: **86115975.4**

(22) Date of filing: **18.11.86**

(54) **Decreasing the viscosity of aqueous carbonaceous fuel slurries to improve atomization.**

(30) Priority: **19.11.85 US 799767**

(43) Date of publication of application:
**03.06.87 Bulletin 87/23**

(45) Publication of the grant of the patent:
**28.03.90 Bulletin 90/13**

(84) Designated Contracting States:
**AT BE CH DE FR GB IT LI NL SE**

(56) References cited:
**US-A- 4 375 358**
**US-A- 4 441 889**

(73) Proprietor: **HENKEL CORPORATION (a Delaware corp.),
7900 West 78th Street, Minneapolis
Minnesota 55435(US)**

(72) Inventor: **Arquette, Robert E., 5619 Hyland Court Drive,
Bloomington, MN 55437(US)**
Inventor: **Maske, Fred J., 1402 W. Iowa Avenue, Falcon
Heights, MN 55108(US)**

(74) Representative: **von Kreisler, Alek, Dipl.-Chem. et al,
Deichmannhaus am Hauptbahnhof, D-5000 Köln 1(DE)**

## Description

BACKGROUND OF THE INVENTION

1. Field of the Invention

Aqueous slurries of solid carbonaceous fuels such as coal are being transported, atomized, and ignited as fuels. Such slurries have had problems caused by the separation of the water and solids due to particle settling. In order to prevent this separation, polymeric stabilizers, gelling agents and viscosity builders are added to the aqueous coal slurries.

2. Statement of the Related Art

U.S. Patent 4,242,098 teaches the addition of water soluble polymers such as xanthan gum and guar gums; guar gum derivatives such as hydroxypropyl guar gum, carboxymethylhydroxypropyl guar gum; and cellulose derivatives such as hydroxyethyl cellulose, and quaternary nitrogen substituted cellulose ethers. A similar teaching is found in U.S. Patent 4,375,358 which teaches that various viscosity builders, gelling agents, and stabilizers are added to solid fuel water slurries for improved stabilization needed during transportation and storage. This patent indicates that biopolymeric water soluble gelling agents such as xanthan gum, cellulose derivatives, and starch derivatives can be used. Preferred materials named are xanthan gum, sodium carboxymethyl cellulose, hydroxypropylated corn starch, hydroxyethyl cellulose, hydroxypropyl methyl cellulose, and methyl cellulose.

The addition of such stabilizers to solid fuel water slurries will prevent water solids separation and allow the slurry to be pumped and stored conveniently without sedimentation. Difficulties occur, however, since the addition of these stabilizers and gelling agents do increase the viscosities of the slurries. U.S. Patent 4,375,358 reports that such an increase in viscosity has a synergistic effect upon stabilization against sedimentation. The viscosity increase, however, makes atomization more difficult. Thus when such fuel slurries are finally used and atomized for combustion the higher viscosity has a detrimental effect on atomization and negatively effects combustion.

A report on coal water slurry evaluations prepared by Babcock and Wilcox Corporation for the Electric Power Research Institute stated that combustion performance is extremely affected by the quality of atomization. Good combustion of coal water slurry requires atomizing the slurry into small droplets which have a high surface area to volume ratio. This allows efficient conversion and stable ignition. As the viscosity of such coal water slurries decreases, good atomization is less difficult to achieve, and atomization improves. The previously mentioned report suggested that the viscosity of a given coal water slurry could be decreased by heating the slurry. This would decrease the viscosity of the particular slurry and thereby improve atomization and combustion performance.

Methods and steps used to decrease the viscosity of solid carbonaceous fuel slurries moreover, must also avoid injury to the coal which would detract from combustion. For this reason, non specific forms of degradation such as chemical degradation of the polymeric stabilizers which serve to increase viscosity is discouraged. Strong acids, which would serve to degrade the stabilizers and thereby decrease the viscosity, would also disadvantageously act upon the solid carbonaceous fuel particles in the slurry. In addition to this, noxious and toxic and corrosive fumes would be produced upon combustion.

It is an object of the instant invention to provide a method which could be used to reduce the viscosity of such polymer stabilized solid carbonaceous fuel water slurries. Other objects will become apparent as this description proceeds.

BRIEF DESCRIPTION

When a solid carbonaceous particle fuel slurry contains at least one polymeric stabilizer, the viscosity can be decreased by using an enzyme to degrade the stabilizer. This process comprises: contacting the polymeric stabilizer with a depolymerase able to degrade the stabilizer and maintaining this contact with the stabilizer under depolymerizing conditions for a sufficient time to degrade an effective amount of the stabilizer. When an effective amount of stabilizer has degraded a decrease in viscosity is achieved. This process can moreover be used to decrease the viscosity to a desired level.

This process can be used with any carbonaceous fuel slurry which contains a polymeric stabilizer. Preferred slurries are aqueous coal slurries containing a sufficient amount of polymeric stabilizer to stabilize and thus increase the viscosity of the slurry. A polymeric stabilizer present in a carbonaceous fuel slurry in a viscosity increasing amount can be degraded with a depolymerase in accordance with the instant process resulting in a decrease in viscosity and a better atomization.

Enzymes are classified according to the type of reaction which is catalyzed. Thus, depolymerase refers to enzymes which are able to "depolymerize" or break apart polymeric molecules. Depolymerase can be subclassified by reference to the reaction mechanism used by the enzyme in depolymerization. Relative to the instant invention, preferred types of depolymerase are: hydrolase depolymerase which depolymerizes by lysis; and oxidoreductase depolymerase which depolymerizes by catalyzing an oxidation reduction reaction. The term "depolymerase" can be applied to material made up of a single enzyme, or two or more enzymes which together perform single steps to accomplish an overall depolymerization. Lyase depolymerase and oxidoreductase depolymerase do not require water as does hydrolase depolymerase. In classifying depolymerase, reference can also be made to the particular substrate upon which the depolymerase acts.

Substantial decreases in viscosity can be achieved by allowing complete enzymatic degradation of the stabilizer. Moderate decreases in viscosity can be achieved by stopping the enzymatic depolymerization of the stabilizer when the desired viscosity level has been achieved. This can be done by such methods as increasing the temperature to destroy the enzyme, varying the pH, or by the addition of a deactivating enzyme selected to stop depolymerization.

Moderate reductions in viscosity can also be achieved by using more than one polymeric stabilizer in the fuel slurry. An enzyme can then be added which will degrade one, but not all of the polymeric stabilizers present.

## DETAILED DESCRIPTION

Any solid particle fuel slurry which is stabilized with polymeric stabilizers can be subjected to the instant process in order to decrease the viscosity. This process can be very advantageously applied to aqueous carbonaceous fuel slurries before atomization. Typically, the slurry is aqueous. The most preferred slurry is coal-water slurry. Coal of varying rank, mineral content, etc. as well as a wide variety of surface active agents are suitable raw materials with which coal-water mixtures can be formulated, stabilized and acted upon by depolymerase.

Fuel slurries containing a sufficient amount of polymeric stabilizer to stabilize the slurry and thus deter particle settling can be subjected to the instant process to lower the viscosity of the slurry before or during storage or before atomization. Even aqueous coal slurries that have as little as .005 parts of polymeric stabilizer per hundred parts of the slurry will have a viscosity reduction when subjected to the instant process. When slurries contain more than one polymeric stabilizer, the instant process can be applied specifically to one of the stabilizers even if this stabilizer is present in a concentration as low as .00025 parts stabilizer per hundred parts slurry. Naturally as the concentration of the stabilizer in the slurry increases, the instant process will have a larger effect in viscosity reduction. References which describe coal water slurries to which the instant process can suitably be applied in order to decrease viscosity are: U.S. Patent 4,242,098 and U.S. Patent 4,441,889.

The instant process can be used to decrease the viscosity of such slurries at any desired time. This process, however is most advantageously done before atomization, although it can also be used before or during storage or even during transportation if desired in a particular case. Any number of references can be obtained which describe these slurries and the stabilizers used in them.

In accordance with the instant invention, depolymerizing enzymes are used to degrade polymeric stabilizers present in aqueous carbonaceous fuel slurries in order to decrease the viscosity of the slurry. The selection of the depolymerase will depend upon the particular polymeric stabilizer present in the slurry. Depolymerase can be selected from commercially available enzymes which are capable of degrading natural, naturally derived or even synthetic polymers.

If desired, a depolymerase can also be developed and obtained using recombinant DNA technology. Such cloning technology can in fact be advantageously employed to provide a depolymerase having a particularly desired characteristic such as stability at higher temperatures. As stated in U.S. Patent 4,237,224 it is practical to introduce genes specifying metabolic or synthetic functions into a microorganism. This reference and others can be obtained showing methods for developing and using recombinant DNA. Two references which describe and discuss recombinant DNA technology and gene cloning are: Genetic Technology, A New Frontier, compiled by the Office of Technology Assessment Publishing Staff, published by Westview Press, Boulder Colorado (1982); and Understanding DNA and Gene Cloning, A guide for the Curious, by Karl Krlica, published by John Wiley and Sons (1984).

Polymers used as stabilizers in solid carbonaceous fuel slurries forming suitable feed material for the instant process include natural and naturally derived stabilizers as: xanthan gum, starch derivatives, cellulose derivatives, polygalactomannan, polygalactomannan derivatives, alginic acid, karaya gum, and carrageenin. Representative of such materials are: carboxymethylcellulose, hydroxypropylated corn starch, hydroxyethylcellulose, hydroxypropyl methyl cellulose, methylcellulose, carboxyl methyl hydroxypropyl guar gum, hydroxypropyl guar gum, quaternary nitrogen substituted cellulose ethers, propoxylated cellulose, methylguar and the like. The instant process can suitably be used with such stabilizers. Preferably, however, the instant process is applied to: (most typically found stabilizers) xanthan gum, guar gum, methyl guar, hydroxypropyl guar gum and carboxymethyl cellulose. Synthetic water soluble polymers which could be added to fuel slurries are: polyacrylamides, polyethylene oxides, and polyvinyl alcohols. Depolymerase can also be used to degrade such synthetic polymers. Preferably the depolymerase is selected from: hydrolase lyase, and oxireductase depolymerase.

The depolymerase is usually selected with regard to the particular stabilizer used in the slurry and with regard to the particular characteristics desired in the enzyme. The depolymerase can achieve depolymerization by a single reaction catalyzed by a group of enzymes, each one designed to accomplish a single step in the polymeric degradation. It is in fact frequently noted even in commercially marketed enzymes, that enzymatic material can actually contain more than one chemically distinct compound each of which could be termed an "enzyme". As previously indicated such enzymatic material is named and classified generally according to the type of reaction catalyzed. Preferred types of depolymerase are hydrolase depolymerase, lyase, depolymerase, and oxidoreductase depolymerase. Suitably, the oxidoreductase depolymerase can include such depolymerizing enzymes as hydroxylases and oxygenases. The lyases can include such depolymerizing enzymes as decarboxylases and aldehyde-lyases.

For some slurries and specific stabilizers it may be necessary or preferred to use more than one enzyme to achieve the desired viscosity reduction. For example, cellulose and cellulose derivatives break down in a series of stages involving more than one enzyme. To achieve a more complete break down of cellulose, and a greater viscosity reduction several enzymes components can be used including: endo glucanases, with or without exoglucanases to remove the glucose unit, cellobiosylhydrolase (removes cellobiose unit), and cellobiase (catalyses hydrolysis of cellobiose). These enzymes can be collectively referred to as cellulase depolymerase or hemicellulose depolymerase.

Alternatively, however, an endo-glucanase can be used to achieve a more moderate viscosity reduction by splitting the cellulose polymer. Depolymerase and stabilizer combinations which can suitably be used with the instant process are: mannase or mannan depolymerase used with polygalactomannan (locust bean gum; guar gum), and polygalactomannan derivatives; and amylase used with starches and starch derivatives.

Conditions needed for the depolymerization of the stabilizing polymers will depend on the specific depolymerase selected. Such conditions needed for enzyme reaction are well known in the art, and are well known for the specific commercially available enzymes.

One of the most important aspects with regard to the instant process is to maintain the temperature of the aqueous slurry below the deactivation temperature of the depolymerase. Generally, enzymes will degrade or begin to degrade at a certain ceiling temperature. For most hydrolytic enzymes, this occurs between 70 and 80°C. In applying the instant process, therefore, the temperature of the slurry should be maintained below 80°C and preferably below 70°C until the desired, or effective degradation has occurred. Acceptably, the temperature should be between 25 and 70°C; preferably between 40 and 65°C. Most preferably, the temperature is maintained at the temperature at which the depolymerase is most active. If it is desired, in a particular case, to maintain the temperature of the slurry at a level higher than 70°C, however, there are some temperature tolerant enzymes which can be selected. In such a case, depending upon the particular enzyme selected, the temperature could be maintained up to 90°C while depolymerization proceeds. As an example, amylase from Bacillus Stearothermophilus is active, and retains 90% of its activity after periods as long as 1 hour at 90°C.

Fuel slurries generally have pH values in the range of from 5-9. Conveniently, most depolymerase is stable and active within this range. The pH of the slurry should be maintained at a point where the depolymerase used is active. Preferably, the pH should be maintained within 1.5 pH units of the the pH value having the highest activity for the particular depolymerase used. Typically, the depolymerase will be active several pH units up and downward from the pH of peak activity. If necessary for a particular depolymerase, an acid or a base can be added to the slurry to adjust the pH value. Carboxylic acids or mineral acids are suitable. Preferred acids are HCl and acidic. Preferred bases are sodium hydroxide, potassium hydroxide, and ammonium hydroxide.

Depolymerization can, if desired, be stopped at a particular viscosity level by: (1) adjusting the pH to a point outside of the active range (2) adjusting the temperature to a point where the depolymerase is inactivated or degraded (denatured); (3) adding an effective amount; preferably at least one equivalent of an enzyme which deactivates the depolymerase. By stopping depolymerization, the viscosity level can be halted at a particular desired point. Deactivating the depolymerase instead of degrading it in order to eliminate it can permit the polymeric degradation to be initiated again by reactivating the enzyme. If desired, however, the depolymerase can be completely eliminated from the slurry by changing the pH or the temperature to a point at which degradation or denaturation of the depolymerase occurs.

Temperatures of the slurry can be brought outside of the active range of the depolymerase in order to stop depolymerization by raising or lowering the temperature. Lowering the temperature to deactivate depolymerization however will result in the corresponding increased viscosity of the aqueous slurry at these lower temperatures. Preferably, therefore, depolymerase is deactivated either by the use of an enzyme designed to deactivate the depolymerizing enzyme or by varying the pH outside of the active range of the depolymerase being used. An increase in temperature however, is preferably used to eliminate depolymerase by degradation or denaturation at the appropriately high temperature. Most enzymes will degrade or become denatured at temperatures in excess of 70°C. Increasing the temperature of the slurry to a point in excess of 70°C and preferably in excess of 75°C for an effective length of time will cause the depolymerase to degrade. The temperature can then be safely lowered without further depolymerization occurring. Thus, if desired, an enzyme which is stable at higher temperatures, can be obtained.

If desired, pH adjustment can be used to stop depolymerization also. The pH can be adjusted to a level above or below the activity range of the particular depolymerase to a point of inactivity. If desired, the pH can also be varied even further to a point higher or lower than the activity range of the depolymerase to a point where the depolymerase will degrade. In this manner, if desired, after viscosity reduction, the depolymerase is substantially eliminated from the slurry by degradation. Advantageously, if the pH or the temperature is adjusted to a point of inactivity of the depolymerase, then depolymerization can be started again merely by bringing the temperature or the pH back within the active range of the depolymerase. The depolymerase will rapidly degrade at pH levels and at temperatures outside the activity and deactivation points. As previously indicated, depolymerase degradation can be accomplished at these levels to eliminate the enzyme. The exact temperature and pH values needed will depend upon the particular depolymerase used.

Depolymerizing conditions are maintained for that length of time sufficient to degrade an effective amount of the polymeric stabilizer. Depolymerization can take place so rapidly that complete depolymerization can be accomplished within a short period of time of between 0.5 minutes and 1 hour by using the optimum pH, temperature, and concentration levels. Although the specific length of time needed for viscosity reduction in each case can be varied by changing such individual factors as the amount of the depolymerase used, the pH, and temperature. When the instant process is being used to decrease the viscosity of a slurry in order to improve atomization or to make atomization easier, the depolymerization is suitably accomplished as the slurry is being pumped to atomization; or when the slurry is temporarily stored (in holding tanks) shortly before atomization. Thus, conveniently, the depolymerase is added to the slurry either in the tank, or during pumping.

The depolymerase should be used in an effective amount relative to the amount of stabilizer present. Acceptably the depolymerase can be used in an amount of from about .001 parts depolymerase per hundred parts of stabilizer (phs) to about 10 phs., preferably in an amount of from about .01 phs to about .5 phs; most preferably from about .1 to 2.5 phs.

Depolymerase generally causes the desired reduction in viscosity by severing the backbone of the polymeric stabilizers thus depolymerizing (or degrading) the polymeric molecule. If desired, however, particular enzymes could be used to sever or shorten the side chains of the polymeric stabilizers. Advantageously, in this regard, side chain severing enzymes can be added in addition to depolymerase. This addition can moreover, allow depolymerase to depolymerize the backbone faster and more completely. Either a hydrolase or lyase can be selected to sever or shorten the particular polymeric side chains. Galactosidase for example, can be used to remove the pendant galactose units from the polymannan backbone of the polygalactomannans.

The polymeric stabilizer is contacted with the depolymerase in any convenient manner. This can be accomplished by the addition of depolymerase to the slurry. For the most immediate results the depolymerase should be in solution when added. If added as a solid it will take time to dissolve before depolymerization will begin. Most conveniently the depolymerase is dissolved and added in an aqueous solution.

The instant invention will be more fully understood from the examples which follow. These examples are intended to clarify the instant invention and not to limit it. All parts and percentages are by weight unless otherwise specified.

## EXAMPLE I

A coal-water mixture was prepared by wet ball milling a mixture of coarsely ground coal, water, nonionic surfactant and a defoamer. This mixture had the following composition: 70% by wt coal (Splash Dam, a product of United Coal Company, Bristol VA); 0.65% by wt non ionic surfactant (Igepal® CO-990 GAF Corporation); and a Trace of defoamer (Colloids® 691, Colloids Inc., Newark NJ).

The ground coal had the following particle size distribution (the percentages indicate the weight percent of the total sample on a moisture free basis which was retained on the designated Tyler screens):

| Tyler Screen Size | % Passing Through Screen |
|---|---|
| 100 | 96.6 |
| 200 | 77.0 |
| 325 | 63.7 |
| 400 | 60.6 |

Using a Brookfield RVT viscometer and a #5 spindle the reading at 20 RPM was 1.5 giving an apparent viscosity of 300 (milli pascal second) (mPa s); a #4 spindle gave a reading of 4.1 for an apparent viscosity of 410. The mixture was used as a base slurry which was split into several aliquot portions. Different polymeric stabilizers were added in a powder form. After complete hydration, dissolution, and mixing of the polymer, the slurry was stabilized, and the viscosity was measured. After this initial viscosity measurement, the depolymerase, cellulase was added to the stabilized coal-water slurry. The coal-water mixture was moderately agitated throughout except when viscosity measurements were made.

5

The table indicates the values obtained from viscosity measurements which were made at 5, 10, 15, 20, 30 and 40 minutes. A final viscosity measurement was made after 16 hours.

The stabilizer used was carboxymethyl cellulose., and the depolymerase was cellulase. The cellulase was in a cellulase preparation marketed by Novo Laboratories, Inc. The cellulase preparation was used in an amount of .6 parts per hundred parts of the coal-water mixture (phm). The activity of this cellulase preparation was 1500 Novo Cellulase Units per gram. Novo has determined this activity and defined the Novo Cellulase Units as follows:

One Novo Cellulase Unit (NCU) is the amount of enzyme which, under standard conditions, degrades carboxy methyl cellulose (CMC) to reducing carbohydrates with a reduction power corresponding to 1 mol glucose per minute.

Standard conditions

Substrate.................CMC (Hercules 7LFD)
Temperature.............................40°C
pH.........................................4.8
Reaction time......................20 minutes

In the table the initial viscosity refers to the viscosity of the stabilized coal-water mixture before the enzyme has been added.

## TABLE I

**VISCOSITY REDUCTION OF STABILIZED COAL-WATER MIXTURES THROUGH THE USE OF ENZYMES**

**VISCOSITY OF UNSTABILIZED COAL-WATER MIXTURE: 300 mPa S; pH = 5.0; AMBIENT TEMPERATURE**

| Stabilizer & Concentration In Parts Per Hundred Coal-Water Mixture (Phm) | Depolymerase Active to Depolymerize The Stabilizer | Initial Viscosity Brookfield RVT #5 Spindle 20 RPM mPa ·S | Viscosity Brookfield RVT, #5 Spindle, 20 RPM Time after addition of enzyme (minutes) | | | | | | |
|---|---|---|---|---|---|---|---|---|---|
| | | | 5 mn | 10 mn | 15 mn | 20 mn | 30 mn | 40 mn | 16 hrs |
| Carboxymethyl-cellulose/.04[1] | Cellulase[2] | 1290[3] | 310[3] | – | – | – | – | – | 230 |
| No polymer | " | 300 | | | | | | | 300 |

[1] Carboxymethylcellulose 9H4F is a product of Hercules Inc., Wilmington DE.

[2] The cellulase preparation used, Celluclast, was a product of Novo Laboratories Inc.

[3] Viscosities determined using a #5 Spindle, 20 RPM

EP 0 224 183 B1

EXAMPLE II

A coal-water mixture was prepared by wet ball milling a mixture of coarsely ground coal, water, nonionic surfactant and a defoamer. This mixture had the following composition: 70% by wt coal (Splash Dam, a product of United Coal Company, Bristol VA); 0.65% by wt non ionic surfactant (Igepal® CO-990 GAF Corporation); and a Trace of defoamer (Colloids® 691, Colloids Inc., Newark NJ).

The ground coal had the following particle size distribution (the percentages indicate the weight percent of the total sample on a moisture free basis which was retained on the designated Tyler screens):

| Tyler Screen Size | % Passing Through Screen |
|---|---|
| 100 | 96.6 |
| 200 | 77.0 |
| .325 | 63.7 |
| 400 | 60.6 |

Using a Brookfield RVT viscometer and a #5 spindle the reading at 20 RPM was 1.5 giving an apparent viscosity of 300 (milli pascal second) (mPa s); a #4 spindle gave a reading of 4.1 for an apparent viscosity of 410. The mixture was used as a base slurry which was split into several aliquot portions. Different polymeric stabilizers were added in a powder form. After complete hydration, dissolution, and mixing of the polymer, the slurry was stabilized, and the viscosity was measured.

After this initial viscosity measurement, the depolymerase was added to the stabilized coal-water slurry. The coal-water mixture was moderately agitated throughout except when viscosity measurements were made.

The table indicates the values obtained from viscosity measurements which were made at 5, 10, 15, 20, 30 and 40 minutes. A final viscosity measurement was made after 16 hours.

The depolymerase and the stabilizers used are listed in the table that follows. The mannanase was contained in an enzyme preparation called Gamanase, a product of Novo Laboratories, Inc. The enzyme preparation was used in an amount of .67 phm. (The preparation also contained hemicellulase).

EP 0 224 183 B1

## TABLE II

### VISCOSITY REDUCTION OF STABILIZED COAL-WATER MIXTURES THROUGH THE USE OF ENZYMES
### VISCOSITY OF UNSTABILIZED COAL-WATER MIXTURE: 300 mPa S; pH = 5.0; AMBIENT TEMPERATURE

| Stabilizer & Concentration In Parts Per Hundred Coal-Water Mixture (Phm) | Depolymerase Active to Depolymerize The Stabilizer | Initial Viscosity Brookfield RVT #5 Spindle 20 RPM mPa S | Viscosity Brookfield RVT, #5 Spindle, 20 RPM Time after addition of enzyme (minutes) | | | | | | |
|---|---|---|---|---|---|---|---|---|---|
| | | | 5 mn | 10 mn | 15 mn | 20 mn | 30 mn | 40 mn | 16 hrs |
| Hydroxypropylguar 0.06 | Mannanase and Hemicellulase[4] | 3440 | 220 | - | - | - | - | - | 100 |
| Methylguar 0.06 | " | 2820 | 180 | - | - | - | - | - | 60 |
| Guar 0.06 | " | 2220 | 180 | - | - | - | - | - | 60 |
| Methylguar 0.036 | " | 320 | 100 | - | - | - | - | - | 80 |
| Xanthan 0.024[5] | " | 2260 | 840 | 780 | 740 | 720 | 720 | 760 | 780 |
| Xanthan/Methylguar (in a wt ratio 4:6 and a concentration of .06 Phm | " | 3280 | 720 | 640 | 660 | 640 | 660 | 680 | 620 |
| No polymer | " | 300 | | | | | | | 300 |

[4](Gamanase®, a product of Novo Laboratories, Inc.) and is supplied as a liquid having an activity of 1,500,000 viscosity Hemicellulase units.
Gamanase is standardized in Viscosity HemiCellulase Units (VHCU), a method based on the reduction of viscosity of a substrate made from locust bean gum.
Standard conditions:

    Substrate........................................locust bean gum
    pH................................................................5.0
    Temperature.....................................................30°C
    Reaction time...............................................60 minutes

[5]Kelzan® S xanthan biopolymer was used, a product of Kelco Inc.

EXAMPLE III

A coal-water mixture was prepared by wet ball milling a mixture of coarsely ground coal, water, nonionic surfactant and a defoamer. This mixture had the following composition: 70% by wt coal (Splash Dam, a product of United Coal Company, Bristol VA); 0.65% by wt non ionic surfactant (Igepal® CO-990 GAF Corporation); and a Trace of defoamer (Colloids® 691, Colloids Inc., Newark NJ).

The ground coal had the following particle size distribution (the percentages indicate the weight percent of the total sample on a moisture free basis which was retained on the designated Tyler screens):

| Tyler Screen Size | % Passing Through Screen |
|---|---|
| 100 | 96.6 |
| 200 | 77.0 |
| 325 | 63.7 |
| 400 | 60.6 |

Using a Brookfield RVT viscometer and a #5 spindle the reading at 20 RPM was 1.5 giving an apparent viscosity of 300 (milli pascal second) (mPa s); a #4 spindle gave a reading of 4.1 for an apparent viscosity of 410. The mixture was used as a base slurry which was split into several aliquot portions. Different polymeric stabilizers were added in a powder form. After complete hydration, dissolution, and mixing of the polymer, the slurry was stabilized, and the viscosity was measured.

After this initial viscosity measurement, the depolymerase was added to the stabilized coal-water slurry. The coal-water mixture was moderately agitated throughout except when viscosity measurements were made.

The table indicates the values obtained from viscosity measurements which were made at 5, 10, 15, 20, 30 and 40 minutes. A final viscosity measurement was made after 16 hours. A solution was prepared which was 5% by weight of the enzyme powder which contained mannanase. This prepared solution was used in an amount of .6 parts per hundred parts of coal-water mixture (phm). The viscosity reduction is shown in the following table.

## TABLE III

### VISCOSITY REDUCTION OF STABILIZED COAL-WATER MIXTURES THROUGH THE USE OF ENZYMES
### VISCOSITY OF UNSTABILIZED COAL-WATER MIXTURE: 300 mPa S; pH = 5.0; AMBIENT TEMPERATURE

| Stabilizer & Concentration In Parts Per Hundred Coal-Water Mixture (Phm) | Depolymerase Active to Depolymerize The Stabilizer | Initial Viscosity Brookfield RVT #5 Spindle 20 RPM mPa S | Viscosity Brookfield RVT, #5 Spindle, 20 RPM Time after addition of enzyme (minutes) | | | | | | |
|---|---|---|---|---|---|---|---|---|---|
| | | | 5 mn | 10 mn | 15 mn | 20 mn | 30 mn | 40 mn | 16 hrs |
| Hydroxy-propylguar 0.06 | Mannanase[6] | 3440 | 2160 | 1880 | 1700 | 1660 | 1700 | 1640 | 860 |
| Methyl Guar 0.06 | " | 2820 | 1380 | 1200 | 1180 | 1060 | 1040 | 1140 | 490 |
| Guar 0.06 | " | 2220 | 1080 | 960 | 940 | 960 | 860 | 860 | 180 |
| No polymer | " | 300 | | | | | | | 300 |

[6] Hemicellulase® 1500, a product of Miles Laboratores. The mannanase was contained in the powdered product.

EP 0 224 183 B1

## Claims

1. A process for decreasing the viscosity of an aqueous carbonaceous fuel slurry containing at least one polymeric stabilizer comprising: contacting the polymeric stabilizer with a sufficient amount of a depolymerase able to degrade at least one of the polymeric stabilizers, and maintaining contact with the polymeric stabilizer under depolymerizing conditions for a time sufficient to degrade an effective amount of the stabilizer, thereby decreasing the viscosity of the slurry.

2. A process as described in claim 1 wherein the depolymerase added to the slurry is in an aqueous solution.

3. A process as described in claim 2 wherein: more than one stabilizer is present in the slurry and wherein the depolymerase is able to degrade at least one but not all of the stabilizers.

4. A process as described in claim 2 wherein: the slurry contains a minimum of one stabilizer selected from the group consisting of: polygalactomannan, a polygalactomannan derivative, cellulose derivatives, starch derivatives, xanthan gum, alginic acid, karaya gum, and carrageenin.

5. A process as described in claim 3 wherein: the slurry contains a cellulose derivative and a stabilizer selected from the group consisting of: polygalactomannan, a polygalactomannan derivative and xanthan, and wherein the depolymerase added will act on the stabilizer selected from the group, but will not act on the cellulose derivative.

6. A process as described in claim 2 wherein the depolymerase is selected from the group consisting of: a hydrolase depolymerase, a lyase depolymerase, and an oxidoreductase depolymerase.

7. A process as described in claim 4 wherein the slurry contains: a polygalactomannan derivative and xanthan gum.

8. A process as described in claim 4 wherein, after viscosity reduction the depolymerase is degraded by changing the pH of the slurry.

9. A process as described in claim 3 wherein the depolymerase is added in an amount of from about .001 to about 10 parts per hundred parts of stabilizer.

10. A process as described in claim 1 wherein the amount of the depolymerase is in the range of from about .001 to about 10 parts per hundred parts stabilizer.

11. A process as described in claim 10 wherein the slurry contains at least one polymeric stabilizer selected from the group consisting of: carboxymethylcellulose, hydroxypropylated corn starch, hydroxyethylcellulose, hydroxypropyl methyl cellulose, methylcellulose, carboxy methyl hydroxypropyl guar gum, hydroxypropyl guar gum, quaternary nitrogen substituted cellulose ethers, propoxylated cellulose, methylguar, xanthan gum, and guar gum.

12. A process as described in claim 11 wherein the slurry is at a pH in the range of from 5 to 9.

13. A process as described in claim 11 wherein, during depolymerization, the slurry is at a temperature between 25 and 70°C.

## Patentansprüche

1. Verfahren zur Erniedrigung der Viskosität einer wenigstens einen polymeren Stabilisator enthaltenden wäßrigen Aufschlämmung eines kohlenstoffhaltigen Brennstoffs, umfassend das In-Berührung-Bringen des polymeren Stabilisators mit einer ausreichenden Menge einer Depolymerase, die zum Abbau wenigstens eines der polymeren Stabilisatoren fähig ist, und das Aufrechterhalten des Kontakts mit dem polymeren Stabilisator unter depolymerisierenden Bedingungen während einer Zeit, die ausreicht, um eine wirksame Menge des Stabilisators abzubauen, wodurch die Viskosität der Aufschlämmung erniedrigt wird.

2. Verfahren nach Anspruch 1, worin die der Aufschlämmung zugesetzte Depolymerase in wäßriger Lösung vorliegt.

3. Verfahren nach Anspruch 2, worin in der Aufschlämmung mehr als ein Stabilisator vorliegt und worin die Depolymerase fähig ist, wenigstens einen, jedoch nicht alle, der Stabilisatoren abzubauen.

4. Verfahren nach Anspruch 2, worin die Aufschlämmung ein Minimum eines Stabilisators enthält, der aus der aus Polygalactomannan, einem Polygalactomannan-Derivat, Cellulose-Derivaten, Stärke-Derivaten, Xanthan-Gum, Alginsäure, Karaya-Gum und Carrageenin bestehenden Gruppe ausgewählt ist.

5. Verfahren nach Anspruch 3, worin die Aufschlämmung ein Cellulose-Derivat und einen Stabilisator enthält, der aus der aus Polygalactomannan, einem Polygalactomannan-Derivat und Xanthan bestehenden Gruppe ausgewählt ist, und worin die zugesetzte Depolymerase auf den aus der Gruppe ausgewählten Stabilisator wirkt, nicht jedoch auf das Cellulose-Derivat.

6. Verfahren nach Anspruch 2, worin die Depolymerase aus der aus einer Hydrolase-Depolymerase, einer Lyase-Depolymerase und einer Oxidoreduktase-Depolymerase bestehenden Gruppe ausgewählt ist.

7. Verfahren nach Anspruch 4, worin die Aufschlämmung ein Polygalactomannan-Derivat und Xanthan-Gum enthält.

8. Verfahren nach Anspruch 4, worin nach der Erniedrigung der Viskosität die Depolymerase durch Änderung des pH-Wertes der Aufschlämmung abgebaut wird.

9. Verfahren nach Anspruch 3, worin die Depolymerase in einer Menge von etwa 0,001 bis 10 Teilen auf 100 Teile des Stabilisators zugesetzt wird.

10. Verfahren nach Anspruch 1, worin die Menge der Depolymerase im Bereich von etwa 0,001 bis 10 Teilen auf 100 Teile des Stabilisators vorliegt.

11. Verfahren nach Anspruch 10, worin die Aufschlämmung wenigstens einen polymeren Stabilisator enthält, der aus der aus Carboxymethylcellulose, hydroxypropylierter Maisstärke, Hydroxyethylcellulose, Hydroxypropylmethylcellulose, Methylcellulose, Carboxymethylhydroxypropyl-Guar-Gum, Hydroxypropyl-Guar-Gum, mit quaternärem Stickstoff substituierten Celluloseethern, propoxylierter Cellulose, Methyl-Guar, Xanthan-Gum und Guar-Gum bestehenden Gruppe ausgewählt ist.

12. Verfahren nach Anspruch 11, worin die Aufschlämmung einen pH-Wert im Bereich von 5 bis 9 hat.

13. Verfahren nach Anspruch 11, worin die Aufschlämmung sich während der Depolymerisation auf einer Temperatur zwischen 25 °C und 70 °C befindet.

**Revendications**

1. Procédé pour abaisser la viscosité d'une suspension aqueuse de combustible carboné contenant au moins un stabilisant polymère comprenant: la mise en contact du stabilisant polymère avec une quantité suffisante d'une dépolymérase capable de dégrader au moins l'un des stabilisants polymères, et le maintien du contact avec le stabilisant polymère dans des conditions de dépolymérisation pendant une durée suffisante pour dégrader une quantité effective du stabilisant, abaissant ainsi la viscosité la suspension.

2. Procédé selon la revendication 1, dans lequel la dépolymérase ajoutée à la suspension est en solution aqueuse.

3. Procédé selon la revendication 2, dans lequel plus d'un stabilisant sont présents dans la suspension et dans lequel la dépolymérase est capable de dégrader au moins l'un des stabilisants mais pas tous.

4. Procédé selon la revendication 2, dans lequel la suspension contient un minimum d'un stabilisant choisi dans le groupe consistant en le polygalactomannane, un dérivé du polygalactomannane, les dérivés cellulosiques, les dérivés de l'amidon, la gomme de xanthane, l'acide alginique, la gomme de sterculia et le carrageenan.

5. Procédé selon la revendication 3, dans lequel la suspension contient un dérivé cellulosique et un stabilisant choisi dans le groupe consistant en le polygalactomannane, un dérivé du polygalactomannane et le xanthane, et dans lequel la dépolymérase ajoutée agit sur le stabilisant choisi dans le groupe, mais n'agit pas sur le dérivé cellulosique.

6. Procédé selon la revendication 2, dans lequel la dépolymérase est choisie dans le groupe consistant en une dépolymérase hydrolase, une dépolymérase lyase et une dépolymérase oxydoréductase.

7. Procédé selon la revendication 4, dans lequel la suspension contient un dérivé du polygalactomannane et de la gomme de xanthane.

8. Procédé selon la revendication 4, dans lequel, après la réduction de la viscosité la dépolymérase est dégradée par modification du pH de la suspension.

9. Procédé selon la revendication 3, dans lequel la dépolymérase est ajoutée en une quantité d'environ 0,001 à environ 10 parties pour cent parties de stabilisant.

10. Procédé selon la revendication 1, dans lequel la quantité dépolymérase est comprise dans la gamme d'environ 0,001 à environ 10 parties pour cent parties de stabilisant.

11. Procédé selon la revendication 10, dans lequel la suspension contient au moins un stabilisant polymère choisi dans le groupe consistant en la corboxyméthylcellulose, l'amidon de mais hydroxypropylé, l'hydroxyéthylcellulose, l'hydroxypropylméthylcellulose, la méthylcellulose, la gomme de carboxyméthylhydroxypropylguar, la gomme d'hydroxypropylguar, les éthers cellulosiques substitués par un azote quaternaire, la cellulose propoxylée, le méthylguar, la gomme de xanthane et la gomme de guar.

12. Procédé selon la revendication 11, dans lequel la suspension est à un pH situé dans la gamme de 5 à 9.

13. Procédé selon la revendication 11, dans lequel, au cours de la dépolymérisation, la suspension est à une température comprise entre 25 et 70°C.